# FASCICULE DE BREVET EUROPEEN

(11) **EP 3 082 565 B1**
(45) Date de publication et mention de la délivrance du brevet: **05.02.2020**
(21) Numéro de dépôt: 14827506.8
(22) Date de dépôt: 12.12.2014
(51) Int. Cl.: A61B 3/032, A61B 3/00, A61B 3/12

(54) **APPAREIL ET PROCÉDÉ DE DÉPISTAGE DES DÉFAUTS DE LA VUE ET DE MESURE D'ACUITÉ VISUELLE D'UN UTILISATEUR**
VORRICHTUNG UND VERFAHREN ZUM SCREENING VON SICHTDEFEKTEN UND ZUR MESSUNG DER SEHSCHÄRFE EINES BENUTZERS
APPARATUS AND METHOD FOR SCREENING FOR DEFECTS IN THE SIGHT AND FOR MEASURING THE VISUAL ACUITY OF A USER

(30) Priorité: 17.12.2013 FR 1362840
(43) Date de publication de la demande: 26.10.2016
(73) Titulaire: Essilor International, 94220 Charenton-le-Pont (FR)
(72) Inventeur: NAUCHE, Michel, 94227 Charenton-le-Pont Cedex (FR); BOUTINON, Stéphane, 94227 Charenton-le-Pont Cedex (FR)
(74) Mandataire: Jacobacci Coralis Harle
(86) Numéro de dépôt international: PCT/FR2014/053316
(87) Numéro de publication internationale: WO 2015/092233

(56) Documents cités:
- EP-A- 0 102 887
- EP-A1- 2 454 988
- US-A1- 2011 025 977
- US-A1- 2013 128 229

## Description

### DOMAINE TECHNIQUE AUQUEL SE RAPPORTE L'INVENTION

La présente invention concerne de manière générale le domaine des appareils et procédés pour l'examen optique des yeux.

Elle concerne plus particulièrement un appareil et un procédé de dépistage des défauts de la vue d'une personne et/ou de mesure d'acuité visuelle.

Ce type d'appareil est destiné à être utilisé dans toutes les situations où un dépistage des défauts de la vue doit être pratiqué. En particulier, un tel appareil est destiné à être utilisé pour le dépistage des défauts de la vue dans le cadre de la médecine du travail, d'un examen du permis de conduire, d'un auto-test en pharmacie par exemple lors de la vente de lunettes de vision de près.

Ce type d'appareil est aussi destiné à être utilisé dans le cadre d'un dépistage visuel et/ou de mesures d'acuité visuelle par un opticien ou un audioprothésiste en magasin.

L'invention concerne en particulier un appareil et un procédé du type à mesure subjective, c'est à dire un appareil de contrôle nécessitant la participation active du patient.

### ARRIERE-PLAN TECHNOLOGIQUE

On connaît notamment du document EP0102887_B1 un appareil d'autotest pour l'examen de la vue des deux yeux d'un patient et pour l'auto-dépistage de ses défaillances visuelles sans intervention d'un opérateur. L'appareil comporte un système optique qui permet la vision d'images-tests portées par une bobine de film présentées suivant trois voies optiques distinctes correspondant respectivement à la vision de près, la vision de loin et la vision intermédiaire. Avantageusement, l'appareil d'autotest comporte un circuit de haut-parleur et un circuit d'enregistrement couplés et/ou synchronisés avec la rotation de la bobine de film, pour donner des instructions et/ou poser des questions au patient et enregistrer des réponses du patient en fonction des images-tests visualisées.

Document EP 2 454 988 A1 révèle un appareil selon le préambule de la revendication indépendante 1.

On connaît aussi un appareil de dépistage des défauts de la vue, sous la dénomination commerciale Ergovision de la société Essilor, qui permet de présenter de tests optiques, ou optotypes, à différentes distances de vision. La personne testée passe de la vision de loin, à la vision intermédiaire puis à la vision de près simplement en abaissant son regard. Un dispositif de synthèse vocale permet de piloter automatiquement l'enchaînement d'une série de tests. Le patient peut répondre aux questions via des boutons d'une télécommande.

Les images-tests ou optotypes utilisés dans ces appareils de dépistage par exemple pour mesurer l'acuité visuelle ou dépister l'hypermétropie sont en général des caractères alphanumériques disposés de manière analogue à une échelle Monoyer de tests optométriques. Ces tests permettent de donner des instructions claires, telles que par exemple : « lisez la ligne n° X », et d'enregistrer des réponses facilement compréhensibles.

Néanmoins, les images-tests de type alphanumérique sont inutilisables avec des personnes illettrées. D'autre part, des biais de mesure d'acuité visuelle peuvent provenir d'une mémorisation des suites de lettres ou de chiffres par le patient ou d'une tendance du patient à deviner plus qu'à ne les visualiser les caractères alphanumériques affichés.

Or, le dépistage des défauts de la vue doit désormais s'effectuer selon la norme ISO8596 qui définit non seulement les distances de présentation d'optotypes mais aussi la forme des optotypes.

Selon cette norme, les optotypes doivent être présentés à une distance correspondant à une vision de loin supérieure à 4 mètres. La distance vision de près n'est pas normalisée mais une plage de distance comprise entre 33 et 50 cm est couramment citée.

De plus, selon la norme ISO8596, les tests de dépistage visuel doivent être basés sur une échelle de Landolt, qui comporte une série d'optotypes identiques, appelés anneaux de Landolt, en forme de cercles ayant une ouverture ou brisure. Le diamètre du cercle varie en fonction de l'acuité visuelle testée. L'orientation de l'ouverture du cercle varie suivant différentes directions qui peuvent être : 0, +45 deg.,+90 deg., +135 deg., +180 deg., +225 deg., +270 deg. et +315 degrés par rapport à une ligne verticale.

L'échelle de Landolt offre l'avantage de pouvoir être utilisée avec des personnes illettrées. De plus, il est difficile à un patient de deviner une zone plus claire ou plus foncée correspondant à l'orientation de l'ouverture d'un anneau de Landolt. En théorie, l'échelle de Landolt permet des mesures d'acuité ne mettant pas en jeu la mémorisation de la morphologie d'une lettre. En pratique, pour une même acuité à tester, c'est-à-dire pour un anneau de Landolt de diamètre déterminé, on présente l'anneau suivant une orientation aléatoire un certain nombre de fois de façon à pouvoir déterminer si la performance visuelle est atteinte ou pas.

Cependant, la réponse orale d'un patient pour indiquer l'orientation d'un anneau de Landolt pose des problèmes de fiabilité. En effet, si les indications haut et bas sont communément assimilées par l'ensemble de la population, la notion de droite et gauche peut être sujette à confusion. Pour les autres directions, telles que + 45 deg., + 135 deg., + 225 deg. et +315 deg., il est en général très difficile de s'assurer de la pertinence de la réponse. Cette utilisation d'une échelle de Landolt peut conduire à une réponse fausse de l'utilisateur, alors que cet utilisateur a détecté visuellement l'orientation correcte de l'anneau.

Il est donc nécessaire de pouvoir enregistrer les réponses du patient à un test basé sur une échelle de Landolt d'une façon sûre en n'introduisant pas de faux positif.

Certains appareils connus présentant des anneaux de Landolt, utilisent un joystick comme interface utilisateur. Si l'utilisation d'un tel accessoire est familière à une population habituée aux jeux vidéo, elle n'est pas totalement spontanée pour un candide.

En général, les appareils de dépistage visuel de l'art antérieur ne permettent pas d'utiliser de manière fiable des images test d'une échelle de Landolt indiquées dans la norme ISO8596.

Un des buts de l'invention est de proposer un appareil et un procédé de dépistage des défauts de la vue d'une personne permettant de présenter dans un volume compact des optotypes, en particulier sous la forme d'anneaux de Landolt, à deux distances de vision très différentes et d'enregistrer une réponse fiable de la personne testée.

### OBJET DE L'INVENTION

Afin de remédier à l'inconvénient précité de l'état de la technique, la présente invention propose un appareil optoélectronique bi-oculaire de dépistage des défauts de la vue et de mesure d'acuité visuelle d'un utilisateur, l'appareil opto-électronique bi-oculaire comportant un boîtier ayant une première ouverture droite et une première ouverture gauche destinées à être disposées respectivement face à l'œil droit et à l'œil gauche de l'utilisateur regardant dans une première direction de visée.

Plus particulièrement, on propose selon l'invention un appareil opto-électronique bi-oculaire de dépistage des défauts de la vue et de mesure d'acuité visuelle comportant les caractéristiques définies dans la revendication indépendante 1.

L'appareil de dépistage permet ainsi à l'aide d'un seul écran d'afficher des optotypes dans un environnement lumineux contrôlé à l'intérieur d'un boîtier et de présenter des informations générales à l'utilisateur, tel qu'un mode d'emploi, une invitation à démarrer ou poursuivre le test de dépistage visuel. Cet appareil permet de modifier les optotypes affichés sans aucun mouvement de composant optomécanique. L'écran de l'appareil de dépistage intègre à la fois les fonctions de source lumineuse et de support physique d'optotypes en nombre illimité.

Selon un mode de réalisation particulier et avantageux, la deuxième zone de l'écran tactile est configurée pour enregistrer des interactions tactiles de l'utilisateur et l'appareil opto-électronique bi-oculaire de dépistage des défauts de la vue et de mesure d'acuité visuelle comporte en outre des moyens électroniques de traitement adaptés pour modifier l'affichage desdits optotypes sur la première partie de la première zone de l'écran en fonction des interactions tactiles enregistrées et/ou pour enregistrer une réponse de l'utilisateur à une première image droite visualisée et/ou à une première image gauche visualisée en fonction desdites interactions tactiles enregistrées.

De façon avantageuse, l'appareil opto-électronique bi-oculaire de dépistage des défauts de la vue et de mesure d'acuité visuelle comporte en outre :
- une deuxième ouverture droite et une deuxième ouverture gauche destinées à être disposées respectivement face à l'œil droit et à l'œil gauche de l'utilisateur regardant dans une deuxième direction de visée ;
- des moyens électroniques adaptés pour afficher une pluralité d'optotypes sur une deuxième partie de la première zone de l'écran tactile, interne au boîtier, lesdites première partie et deuxième partie de la première zone de l'écran tactile étant séparées spatialement ; et
- le système optique étant configuré pour former une deuxième image droite de la deuxième partie de la première zone de l'écran tactile en direction de la deuxième ouverture droite à une deuxième distance de vision suivant un troisième chemin optique, et le système optique étant configuré pour former une deuxième image gauche de la deuxième partie de la première zone de l'écran tactile en direction de la deuxième ouverture gauche à ladite deuxième distance de vision suivant un quatrième chemin optique.

Selon un mode de réalisation particulier et avantageux, l'appareil opto-électronique bi-oculaire de dépistage des défauts de la vue et de mesure d'acuité visuelle comporte en outre :
- une troisième ouverture droite et une troisième ouverture gauche destinées à être disposées respectivement face à l'œil droit et à l'œil gauche de l'utilisateur regardant dans une troisième direction de visée ; et
- le système optique étant configuré pour former une troisième image droite de la troisième partie de la première zone de l'écran tactile en direction de la troisième ouverture droite à une troisième distance de vision suivant un cinquième chemin optique, et le système optique étant configuré pour former une troisième image gauche de la troisième partie de la première zone de l'écran tactile en direction de la troisième ouverture gauche à ladite troisième distance de vision suivant un sixième chemin optique.

D'autres caractéristiques non limitatives et avantageuses d'un appareil opto-électronique bi-oculaire de dépistage des défauts de la vue et de mesure d'acuité visuelle conforme à l'invention sont les suivantes :
- l'écran tactile est disposé dans l'appareil opto-électronique bi-oculaire de manière à présenter une troisième zone externe destinée à servir d'interface à un opérateur, ladite troisième zone externe étant séparée spatialement desdites première zone et deuxième zone de l'écran tactile ;
- la pluralité d'optotypes comporte un anneau de Landolt ayant une orientation et un diamètre déterminés et les moyens de traitement sont adaptés pour indiquer l'orientation ou pour modifier l'orientation et/ou le diamètre de l'anneau de Landolt par interaction de l'utilisateur via la deuxième zone externe de l'écran tactile ;
- la pluralité d'optotypes comporte un caractère alphanumérique ayant une taille déterminée et les moyens de traitement sont adaptés pour désigner un caractère alphanumérique parmi un ensemble de caractères alphanumériques ou pour modifier le caractère alphanumérique affiché et/ou la taille du caractère alphanumérique affiché par interaction de l'utilisateur via la deuxième zone externe de l'écran tactile ;
- la première zone de l'écran tactile, interne au boîtier, est adaptée pour former un moyen d'éclairage adapté pour éclairer un optotype de mesure de réserve d'accommodation ou pour éclairer l'œil droit et/ou gauche de l'utilisateur, de préférence pendant l'affichage d'un optotype ;
- l'écran tactile est amovible du boîtier de l'appareil.

Selon un mode de réalisation particulier et avantageux, l'appareil opto-électronique bi-oculaire de dépistage des défauts de la vue et de mesure d'acuité visuelle comporte en outre :
- des moyens électroniques de traitement adaptés pour modifier l'affichage de la pluralité d'optotypes dans la première zone de l'écran tactile en fonction des interactions de l'utilisateur enregistrées via la deuxième zone externe de l'écran tactile ;
- des moyens d'enregistrement d'une interaction de l'utilisateur via la deuxième zone externe de l'écran tactile et dans lequel les moyens électroniques de traitement sont adaptés pour déduire de cet enregistrement une mesure d'acuité visuelle de l'œil droit et/ou de l'œil gauche pour la première distance de vision, respectivement pour la deuxième distance de vision et/ou pour la troisième distance de vision, la première distance de vision correspondant de préférence une distance de vision de près inférieure ou égale à 50 cm, la deuxième distance de vision correspondant de préférence une distance de vision de loin supérieure ou égale à 4 mètres, la troisième distance de vision correspondant de préférence une distance de vision intermédiaire comprise entre 50 cm et 3 m ;
- des moyens d'obturation adaptés pour obturer sélectivement une première, deuxième et/ou troisième ouverture droite et/ou une première, deuxième et/ou troisième ouverture gauche, lesdits moyens d'obturation comprenant par exemple des moyens d'affichage spatialement sélectifs sur l'écran tactile.;
- des moyens de modification de la luminance et/ou du contraste des images affichées sur la première zone de l'écran tactile ;
- des moyens de mesure du punctum proximum d'accommodation de l'utilisateur ;
- la première zone de l'écran tactile, interne au boîtier, est adaptée pour former un moyen d'éclairage adapté pour éclairer un optotype de mesure du punctum proximum d'accommodation ;
- l'écran tactile est amovible du boîtier de l'appareil.

L'invention propose également un procédé de dépistage des défauts de la vue et de mesure d'acuité visuelle d'un utilisateur, le procédé mettant en œuvre un appareil opto-électronique bi-oculaire selon l'une des revendications 1 à 14 et comprenant les étapes suivantes :
a. affichage d'une pluralité d'optotypes sur la première zone de l'écran tactile, ladite pluralité d'optotypes comprenant au moins un anneau de Landolt, ayant un diamètre et une ouverture d'orientation prédéterminée ;
b. modification du diamètre et/ou d'une indication de l'orientation de l'ouverture de l'anneau de Landolt détectée visuellement par interaction via la deuxième zone de l'écran tactile ;
c. validation, via la deuxième zone de l'écran tactile, d'un diamètre et/ou d'une indication de l'orientation de l'anneau de Landolt détectée visuellement par l'utilisateur.

Enfin, l'invention propose un procédé de dépistage des défauts de la vue et de mesure d'acuité visuelle d'un utilisateur, le procédé mettant en œuvreun appareil opto-électronique bi-oculaire selon l'une des revendications 1 à 14 et comprenant les étapes suivantes :
a. affichage d'une pluralité d'optotypes sur la première zone de l'écran tactile, ladite pluralité d'optotypes comprenant au moins un caractère alphanumérique ayant une taille prédéterminée ;
b. modification du caractère alphanumérique affiché, de la taille du caractère alphanumérique affiché et/ou d'une indication du caractère alphanumérique par interaction via la deuxième zone de l'écran tactile ;
c. validation par l'utilisateur, via la deuxième zone de l'écran tactile, d'une taille et/ou d'une indication du caractère alphanumérique affiché.

### DESCRIPTION DETAILLEE D'UN EXEMPLE DE RÉALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente schématiquement une vue externe en perspective d'un appareil de dépistage visuel selon un mode de réalisation de l'invention ;
- la figure 2 représente une vue du côté de l'utilisateur d'un appareil de dépistage visuel selon une variante de la figure 1 ;
- la figure 3 représente une vue de dessus de l'écran d'un appareil de dépistage visuel selon un exemple de réalisation de l'invention ;
- la figure 4 représente une vue en coupe illustrant le système opto-électronique d'un appareil de dépistage visuel selon une première variante de l'invention ;
- la figure 5 représente une vue interne en perspective illustrant les chemins optiques dans un appareil de dépistage visuel selon une autre variante de l'invention ;
- la figure 6 illustre une interface graphique pour indiquer la position de l'ouverture d'un anneau de Landolt selon un mode de réalisation du procédé de l'invention ;
- la figure 7 illustre une interface graphique pour indiquer un symbole alphanumérique affiché selon un autre mode de réalisation du procédé de l'invention.

### Dispositif

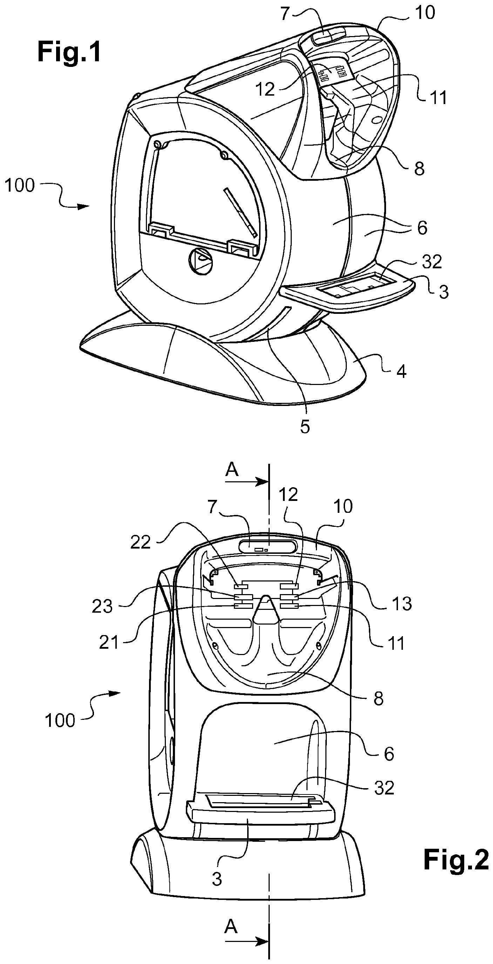

Sur les figures 1-2, on a représenté des vues externes d'un appareil de dépistage visuel 100 selon deux variantes d'un mode de réalisation de l'invention.

L'appareil de dépistage visuel 100 illustré sur les figures 1-2 se présente sous la forme d'un appareil compact qui peut être posé sur une table. L'appareil de dépistage visuel 100 comporte un socle 4 et un boîtier 10, composé d'un ou de plusieurs éléments, qui entoure le système opto-électronique interne à l'appareil. Le boîtier 10 sert à isoler optiquement le système opto-électronique interne de l'éclairement ambiant.

De façon particulièrement avantageuse, l'appareil comporte une zone d'appui frontal 7 et/ou une mentonnière 8 pour maintenir la tête de l'utilisateur dans une position fixe, quelle que soit la direction de l'axe de visée du regard.

En option, l'appareil 100 comporte un rail de guidage 5 qui permet d'incliner le boîtier 10 par rapport au socle 4, de manière à adapter l'appareil à la taille de l'utilisateur pour le confort des tests.

La figure 2 représente le côté de l'appareil de dépistage visuel 100 qui est disposé face à l'utilisateur souhaitant tester son acuité visuelle ou ses défauts de la vue. Le boîtier 10 présente une première ouverture droite 11 et une première ouverture gauche 21 destinées à être disposées respectivement face à l'œil droit et à l'œil gauche de l'utilisateur regardant dans une première direction de visée. De préférence, la première direction de visée, correspondant à la vision de près, est inclinée d'environ 30 degrés par rapport à un plan horizontal passant par le centre de rotation optique des yeux de l'utilisateur.

L'appareil de dépistage visuel 100 comporte un écran 30.

L'écran 30 a une première zone 31, qui est disposée à l'intérieur du boîtier et qui n'apparaît donc pas sur les figures 1-2 et une deuxième zone 32 disposée à l'extérieur du boîtier 10. Selon l'invention, le boîtier comporte une paroi externe 6 opaque qui est disposée transversalement à la surface de l'écran de manière à séparer la première zone 31 de la deuxième zone 32. La paroi externe 6 permet à un utilisateur d'accéder à la deuxième zone 32 pour des interactions visuelles et/ou tactiles. Au contraire, la paroi externe 6 empêche d'accéder directement à la première zone 31 et forme un obturateur qui empêche l'utilisateur de visualiser la première zone 31, lorsque l'écran est en place dans l'appareil de dépistage 100.

Dans un mode de réalisation illustré sur la figure 1, l'écran est un écran plat, par exemple de type écran LCD équipé d'une dalle tactile qui est intégré à l'appareil 100. Dans ce cas, l'appareil de dépistage comporte une électronique de commande de l'écran plat. Dans l'exemple illustré sur la figure 1, l'écran 3 est intégré à l'appareil de dépistage 100 et ne peut être séparé de l'appareil qu'en démontant l'appareil.

Dans un autre mode de réalisation illustré sur les figures 2-3, l'écran est avantageusement un écran de tablette tactile amovible. La tablette tactile 3 intègre une alimentation électrique, une électronique de commande permettant le pilotage de l'écran, une électronique de communication avec un ou plusieurs dispositifs externes de type télécommande, un ordinateur de contrôle pour la conduite et l'enregistrement des résultats de tests de dépistage visuel ou encore une imprimante, pour l'édition d'un rapport de tests de dépistage visuel.

Dans l'exemple illustré sur la figure 2, la tablette tactile 3 est insérée dans un logement de l'appareil et cette tablette est facilement amovible.

Dans une variante, l'appareil de dépistage comporte un dispositif de synthèse vocale configurée pour émettre des messages audio d'explication complémentaire en fonction des tests de dépistage visuel affichés sur l'écran. Les messages audio émis par synthèse vocale complètent ainsi les messages visuels affichés sur l'écran.

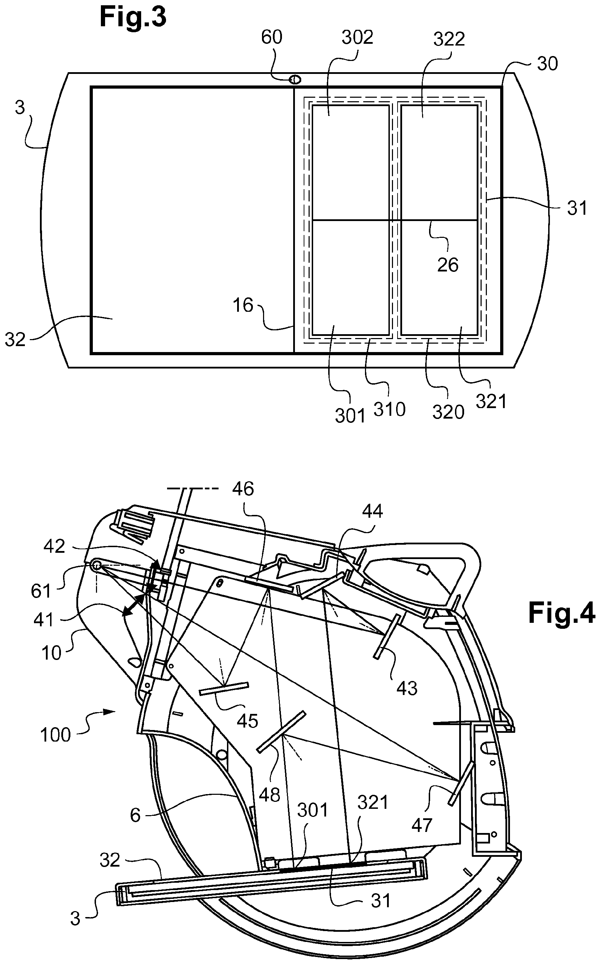

La figure 3 représente une vue de dessus de l'écran d'un appareil de dépistage visuel selon un exemple de réalisation de l'invention.

L'écran comporte une première zone 31 et une deuxième zone 32 qui sont séparées spatialement. Par exemple, l'écran est un écran de tablette de 10,1 pouces de diagonale ayant une résolution d'environ 2560 x1600 pixels, soit une résolution de 300dpi. La première zone 31 et la deuxième zone 32 occupent chacune respectivement environ une moitié de la surface de l'écran divisé dans le sens de sa longueur. Par exemple, une extrémité de la paroi 6 externe du boîtier de l'appareil est placée le long d'une ligne 16 qui sépare la première zone 31 de la deuxième zone 32.

La première zone 31 est ainsi disposée face à un système optique à l'intérieur du boîtier de l'appareil. Un exemple de système optique est décrit en détail dans le présent document en lien avec les figures 4-5.

La première zone 31 de l'écran située à l'intérieur de l'appareil est configurée de façon à présenter les optotypes souhaités. L'éclairage émis par l'écran permet de former des images lumineuses sans source de lumière additionnelle. Avantageusement, la modulation de l'intensité de l'image émise par l'écran permet d'adapter la luminance des images en fonction des conditions souhaitées : éclairage normal de jour, éclairage de faible ou de forte intensité.

De façon particulièrement avantageuse, l'appareil est configuré pour tester la vision de l'utilisateur à plusieurs distances de vision, par exemple la vision de près et la vision de loin. Dans ce cas, la première zone 31 peut être divisée en une première partie 310 destinée à l'affichage d'optotypes pour une première distance de vision, par exemple en vision de près, et en une deuxième partie 320 destinée à l'affichage d'optotypes pour une deuxième distance de vision, par exemple en vision de loin. Par ailleurs, la première partie 310 peut aussi être divisée par une ligne 26 en une première partie droite 301 de l'écran disposée face à un premier chemin optique formant une image en direction de l'œil droit de l'utilisateur et respectivement une première partie gauche 302 de l'écran disposée face à un deuxième chemin optique, séparé du premier chemin optique, formant une image en direction de l'œil gauche de l'utilisateur. De même, la deuxième partie 320 peut aussi être divisée par la ligne 26 en une deuxième partie droite 321 de l'écran disposée face à un troisième chemin optique formant une image en direction de l'œil droit de l'utilisateur et respectivement une deuxième partie gauche 322 de l'écran disposée face à un quatrième chemin optique formant une image en direction de l'œil gauche de l'utilisateur.

La deuxième zone 32 externe de l'écran est ainsi disposée à proximité de l'utilisateur ou du patient. La deuxième zone 32 de l'écran peut avantageusement être utilisée comme écran d'accueil permettant la sélection du ou des tests souhaités. Cette deuxième zone 32 externe assure la fonction d'interface graphique et/ou tactile avec l'utilisateur et sert à enregistrer les réponses du patient aux tests de dépistage.

Dans le cas d'une utilisation en autotest, la deuxième zone 32 externe de l'écran peut servir à afficher un mode d'emploi ou une invitation à dépister sa vue en totale autonomie.

L'appareil de dépistage offre ainsi l'avantage de présenter des tests de dépistage visuel et d'enregistrer directement les réponses du patient sur un seul et même écran. L'écran de l'appareil de dépistage 100 intègre à la fois les fonctions de source lumineuse, de support physique d'optotypes en nombre illimité et d'interface avec l'utilisateur.

La figure 4 représente schématiquement une vue en coupe AA d'un appareil de dépistage visuel tel que représenté sur la figure 2.

L'appareil de dépistage 100 de la figure 4 comporte un boîtier 10 et une tablette tactile 3. Selon l'invention, une paroi externe 6 permet de séparer physiquement et optiquement l'écran de la tablette tactile 3 en une première partie 31 située à l'intérieur de l'appareil et une deuxième partie 32 accessible à l'extérieur de l'appareil 100, par exemple à un utilisateur.

L'appareil de dépistage 100 comporte un système optique arrangé de manière à former une image de la première zone 31 de l'écran à différentes distances de vision en fonction de la direction de visée.

Dans l'exemple illustré sur la figure 4, le système optique comporte des lentilles et des miroirs.

Plus précisément, le système optique comporte différents chemins optiques associés respectivement à différentes distances de vision.

L'utilisateur place sa tête contre la zone d'appui frontal 7 et/ou la mentonnière 8. De cette manière, l'œil droit, respectivement l'œil gauche, de l'utilisateur se trouve face aux ouvertures droites 11, 12, 13, respectivement face aux ouvertures gauches 21, 22, 23 de l'appareil de dépistage 100.

Sans bouger la tête, par un simple abaissement du regard, l'utilisateur incline la direction de son regard soit vers les premières ouvertures droite 11 et/ou gauche 21 pour un test en vision de près, soit vers les deuxièmes ouvertures droite 12 et/ou gauche 22 pour un test en vision de loin ou soit vers les troisièmes ouvertures droite 13 et/ou gauche 23 pour un test en vision à une distance intermédiaire.

A titre d'exemple, on a représenté sur la figure 4 le centre de rotation optique 61 de l'œil droit. Pour un angle d'abaissement du regard compris entre 0 et 10 degrés par rapport à un plan horizontal, l'axe de visée du regard de l'utilisateur passe par la troisième ouverture droite 13 pour l'œil droit, et/ou, respectivement, par la troisième ouverture gauche 23 pour l'œil gauche. Pour un angle d'abaissement supplémentaire du regard compris entre 10 et 15 degrés, l'axe de visée du regard de l'utilisateur passe par la deuxième ouverture droite 12 pour l'œil droit, et/ou, respectivement, par la deuxième ouverture gauche 22 pour l'œil gauche. Enfin, pour un angle d'abaissement du regard encore supplémentaire d'environ 10 à 15 degrés, l'axe de visée du regard de l'utilisateur passe par la première ouverture droite 11 pour l'œil droit, et/ou, respectivement, par la première ouverture gauche 21 pour l'œil gauche.

Un premier chemin optique est associé à la vision de près pour l'œil droit et comprend une lentille 41, disposée à proximité de la première ouverture 11, un miroir plan 45, un miroir plan 46 et une lame semi-transparente 48. La lentille 41 forme une image d'une première partie 301 de la première zone 31 de l'écran. Un ou plusieurs optotypes sont affichés sur la première partie 301 de la première zone 31 de l'écran pour un test de dépistage en vision de près. L'image émise par la première partie 301 de l'écran est transmise à travers la lame semi-réfléchissante 48, réfléchi par le miroir plan 46 puis par le miroir plan 45 et enfin transmis par la lentille 41 en direction du centre de rotation optique 61 de l'œil droit via la première ouverture 11. La lentille 41 est disposée de manière à former une image virtuelle de la première partie 301 de l'écran à une distance optique inférieure ou égale à 50 cm, qui correspond à la distance de vision de près.

Un deuxième chemin optique est associé à la vision de près pour l'œil gauche et comprend des composants optiques analogues et disposés symétriquement par rapport au plan AA de manière à former une image virtuelle de la première partie 302 de l'écran en direction de la première ouverture gauche 21.

Un troisième chemin optique est associé à la vision de loin pour l'œil droit et comprend une lentille 42, disposée à proximité de la deuxième ouverture 12, un miroir plan 43 et un miroir plan 44. La lentille 42 forme une image d'une deuxième partie 321 de la première zone 31 de l'écran. Un ou plusieurs optotypes sont affichés sur la deuxième partie 321 de la première zone 31 de l'écran pour un test de dépistage en vision de loin. L'image émise par la deuxième partie 321 de l'écran est réfléchi sur le miroir plan 44 puis sur le miroir plan 43 et enfin transmis par la lentille 42 en direction du centre de rotation optique 61 de l'œil droit via la deuxième ouverture 12 face à l'œil droit dans l'axe de visée de loin. La lentille 42 est disposée de manière à former une image virtuelle de la deuxième partie 321 de l'écran à une distance optique supérieure ou égale à 4 mètres, qui correspond à la distance de vision de loin.

Un quatrième chemin optique est associé à la vision de loin pour l'œil gauche et comprend des composants optiques analogues (ouverture 22, lentille 42, miroirs 43, 44) disposés symétriquement par rapport au plan AA de manière à former une image virtuelle de la deuxième partie 322 de l'écran en direction de la deuxième ouverture gauche 22.

De façon avantageuse, un cinquième et un sixième chemins optiques associés à la vision intermédiaire comprennent un miroir plan 47 et la lame-semi-réfléchissante 48. Un ou plusieurs optotypes sont affichés sur la première partie 301 de la première zone 31 de l'écran pour un test de dépistage en vision intermédiaire. L'image émise par la première partie 310 de l'écran est réfléchi sur la lame-semi-réfléchissante 48 puis sur le miroir plan 47. Ainsi, le cinquième chemin optique forme l'image de la première partie 301 de l'écran via la troisième ouverture droite 13 sur l'œil droit et respectivement, le sixième chemin optique forme l'image de la première partie 302 de l'écran via la troisième ouverture gauche 23 sur l'œil gauche. Les cinquième et sixième chemins optiques passent par exemple entre les lentilles 41 et 43. Le cinquième ou sixième chemin optique permet à l'œil de l'utilisateur de former une image de la première partie 301, 302 de l'écran à une distance optique comprise entre 50 cm et 3 mètres, qui correspond à la distance de vision intermédiaire.

Le système optique de l'appareil de dépistage visuel est fixe : il ne nécessite aucun mouvement de composant optique pour modifier la direction de visée du regard ni la distance de vision des images d'optotypes. La position de l'écran reste ainsi fixe et indépendante de la proximité optique des tests pour les différentes valeurs de proximité. Cette configuration optique confère l'appareil de dépistage visuel une grande robustesse.

Les miroirs plans de renvoi 43, 44, 45, 46, 47 ainsi que la lame semi-réfléchissante 48 permettent de replier les différents chemins optiques de manière à rendre l'appareil de dépistage très compact.

La lame semi-réfléchissante 48 permet de manière passive d'utiliser une même partie 310 de la première zone de l'écran pour former des images d'optotypes à deux distances de vision distinctes et suivant deux directions de visée du regard distinctes. Un obturateur mobile (non représenté) permet de sélectionner un chemin optique correspondant à l'une ou l'autre des deux distances de vision.

Dans un mode de réalisation tel que détaillé ci-dessus, le système optique comporte un chemin optique droit tel que détaillé ci-dessus et un chemin optique gauche, symétrique du chemin optique droit par rapport au plan de la figure 4, pour former différentes voies optiques entre d'une part les première, deuxième et troisième ouvertures gauches 21, 22 et 23 et d'autre part la première partie gauche 302 et la deuxième partie gauche 322 de l'écran. Les lentilles 41, 42 utilisées sont de préférence des composants optiques distincts sur les chemins optiques droit et gauche. Les miroirs 43-47 et la lame semi-transparente utilisés sur les chemins optiques droit et gauche peuvent être soit des composants distincts soit constitués de composants s'étendant de part et d'autre du plan de symétrie AA.

Dans un autre mode de réalisation, les mêmes composants optiques permettent simultanément de former différentes voies optiques entre d'une part les première, deuxième et troisième ouvertures gauches 21, 22 et 23 et d'autre part la première partie de l'écran 31, et plus précisément la première partie gauche 302 et la deuxième partie gauche 322 de l'écran.

Par un simple abaissement du regard, l'utilisateur peut visualiser des images d'optotypes dans des directions de visée correspondant à l'angle d'abaissement naturel du regard respectivement en vision de près, de loin et en vision intermédiaire et à des distances respectives correspondant aux distances indiquées par exemple dans la norme ISO8596.

De préférence, l'appareil comporte des moyens pour isoler optiquement la voie optique droite de la voie optique gauche aux différentes distances de vision. Les moyens d'isolation optique comprennent par exemple une cloison interne opaque disposée dans un plan vertical de symétrie (par exemple le plan de coupe AA sur la figure 2) de l'appareil de dépistage 100. Cette cloison interne permet d'éviter des réflexions internes parasites. Les moyens d'isolation optique peuvent aussi comprendre des diaphragmes disposés à proximité d'un ou de plusieurs miroirs 43-47.

De manière alternative et/ou complémentaire, l'appareil de dépistage comporte un ou plusieurs obturateurs permettant d'obturer sélectivement une ou plusieurs des ouvertures 11, 12, 13, 21, 22, 23. Ces obturateurs permettent de limiter l'étendue du champ de vision dans une direction de visée.

Pour un test en vision monoculaire, on utilise les capacités d'affichage sélectives de l'écran pour former une image sur une partie prédéterminée de l'écran 301, 302, ou 321, 322.

De façon particulièrement avantageuse, le système optique comporte des prismes de convergence disposés devant les ouvertures droite et gauche en particulier pour compenser la convergence du regard en vision de près et/ou en vision intermédiaire. L'écran 31 et le système optique forment une image pour chaque œil à une distance de vision déterminée. Les prismes de convergence permettent aux deux yeux de converger à la distance de présentation. Ainsi, l'utilisateur peut fusionner les deux images, droite et gauche, à la distance de vision choisie.

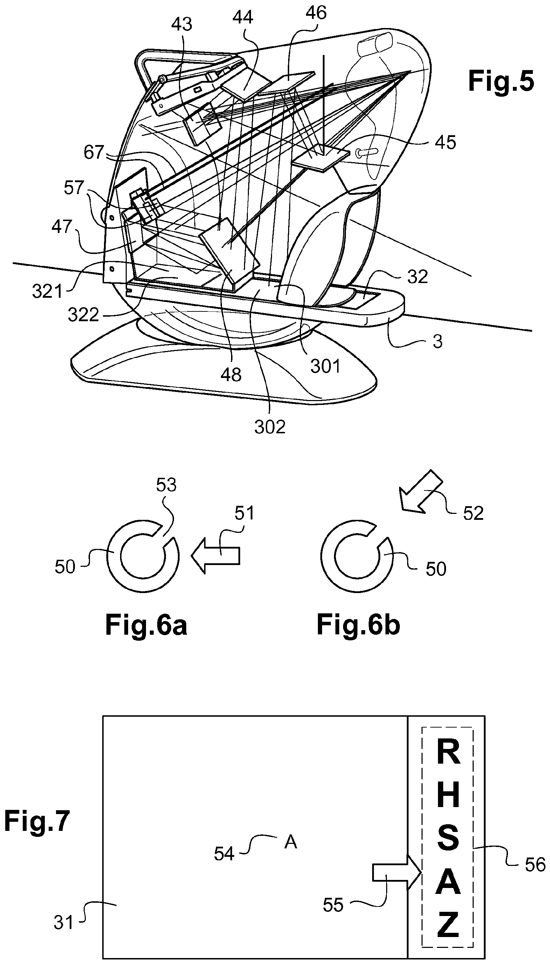

La figure 5 représente schématiquement les différents chemins optiques selon une variante d'un appareil de dépistage visuel.

Les mêmes signes de référence désignent les mêmes éléments que sur la figure 4.

Dans la variante de la figure 5, les premier et deuxième chemins optiques correspondant à la vision de près partent de la première partie de l'écran droite 301 et respectivement gauche 302, se réfléchissent sur le miroir plan 46, sur le miroir plan 45 et sont transmis dans la direction de visée correspondant à un abaissement du regard d'environ 30 degrés. Les troisième et quatrième chemins optiques correspondant à la vision de loin partent de la deuxième partie de l'écran droite 321 et respectivement gauche 322, sont transmis par la lame semi-réfléchissante 48, réfléchis sur le miroir plan 44, sur le miroir plan 43 et sont transmis dans la direction de visée correspondant à un abaissement du regard d'environ 15 degrés. Enfin, les cinquième et sixième chemins optiques correspondant à la vision intermédiaire partent de la deuxième partie de l'écran droite 321 ou gauche 322, sont réfléchis par la lame semi-réfléchissante 48, puis sur le miroir plan 47 dans la direction de visée correspondant à un abaissement du regard d'environ 25 degrés. Ainsi, dans cette variante, la lame semi-réfléchissante 48 sépare les chemins optiques de la vision de loin et de la vision intermédiaire. Par comparaison, dans le mode de réalisation illustré sur la figure 4, la lame semi-réfléchissante 48 sépare les chemins optiques de la vision de près et de la vision intermédiaire.

Les dimensions des miroirs sont sélectionnées en fonction de leur position pour permettre à l'utilisateur de disposer d'un champ de vision confortable suivant le chemin optique de chaque œil et dans chaque axe de visée du regard.

De manière optionnelle, l'appareil de dépistage de la figure 5 comporte en outre un sous-ensemble de mesure de la réserve d'accommodation du patient, aussi appelée punctum proximum. De manière connue par ailleurs, une mesure de réserve d'accommodation vise à déterminer la réserve d'accommodation de l'utilisateur, c'est-à-dire la distance minimum de vision nette à courte distance. Cette mesure permet notamment de dépister une presbytie naissante.

Dans l'exemple représenté sur la figure 5, le sous-ensemble de mesure de la réserve d'accommodation comporte deux porte-test 57, droit et gauche, mobiles en translation suivant des rails de guidage 67 disposés parallèlement à l'axe de vision intermédiaire. Une motorisation codée permet par exemple de modifier la distance optique entre les porte-tests 57 et l'utilisateur dans la direction de visée intermédiaire. Avantageusement, le porte-test 57 est éclairé par le système d'éclairage de l'écran, par exemple par rétro-éclairage. De préférence, le sous-ensemble de mesure de la réserve d'accommodation est monté sur un dispositif d'escamotage combiné au mouvement de translation de façon à pouvoir libérer le champ de vision lorsque que ce sous-système n'est pas utilisé. Par exemple, le dispositif d'escamotage comporte deux pignons situés de part et d'autre de l'axe de translation du sous-ensemble. Lorsque le sous-ensemble arrive en fin de course du mouvement de translation, les deux pignons viennent s'engrener sur deux crémaillères disposées en vis-à-vis. Un mouvement de rotation des deux porte-test 57 s'opère jusqu'à l'escamotage final des porte-tests 57.

La réduction de la luminosité de l'image émise par l'écran éventuellement associé à un filtre peut être utilisée de façon à évaluer l'acuité visuelle dans des conditions de vision mésopique, c'est-à-dire de très faible éclairement ambiant.

Dans un mode de réalisation non illustré sur les figures, une troisième zone de l'écran 30 est accessible et visible par un opérateur, mais pas par l'utilisateur. Par exemple, la troisième zone de l'écran est disposée à l'opposé de la première zone de l'écran disposée face à l'utilisateur. Dans un exemple de réalisation, la première zone est accessible à l'extérieur du boîtier, sur un côté de l'appareil de dépistage et la troisième zone de l'écran 30 est accessible à l'extérieur du boîtier sur un côté opposé de l'appareil de dépistage, la deuxième zone restant inaccessible et invisible à l'intérieur du boîtier. Cette troisième zone permet à un opérateur ou à un opticien de piloter le déroulement d'une séquence de tests de dépistage et/ou de suivre le déroulement des tests d'un utilisateur.

L'appareil de dépistage peut se décliner en version de base avec uniquement un écran tactile accessible à l'utilisateur de manière autonome et en version plus complète avec un dispositif de synthèse vocale pour assister l'utilisateur dans les d'auto- tests.

L'appareil de dépistage peut aussi faire intervenir un opérateur ayant accès à une zone de l'écran qui n'est pas accessible à l'utilisateur, ou ayant accès à une interface reliée à l'appareil de dépistage par des moyens de télécommunication filaire ou sans fil, cette interface comprenant par exemple un ordinateur de contrôle dont l'écran duplique la première zone de l'écran de l'appareil de dépistage et des boutons de commande.

Différentes options peuvent être intégrées à l'appareil de dépistage dans le but d'effectuer d'autres que les tests d'acuité visuelle ou de modifier les conditions dans lesquelles sont réalisés les tests d'acuité visuelle.

Comme indiqué plus haut, la lumière émise par l'écran suffit pour former une image de tests, dans une gamme de luminance correspondant un éclairement de jour.

En option, l'appareil de dépistage comporte des éléments additionnels pour réaliser par exemple un test d'éblouissement, un test d'acuité en contraste variable ou une mesure d'acuité en vision mésopique.

Pour la réalisation d'un test d'éblouissement, l'appareil intègre une source lumineuse supplémentaire placée dans le champ de vision de l'utilisateur lorsque celui-ci regarde à travers une des ouvertures. La source lumineuse supplémentaire est sélectionnée de manière à émettre une luminance pendant environ une dizaine de secondes pour éblouir le patient et générer un scotome de taille suffisante par rapport au test présenté. Ce dispositif permet ainsi d'analyser la récupération de la fonction visuelle de l'utilisateur après un éblouissement.

Pour la réalisation d'un test d'acuité en contraste variable, on implémente dans l'appareil de dépistage une mesure de performance de l'acuité visuelle du patient dans des conditions standard de luminance, avec des optotypes noirs sur fond blanc affichés avec le contraste maximum en fonction de spécificités de l'écran utilisé. Puis, on modifie le niveau de contraste des optotypes, par exemple en réduisant le contraste des optotypes de 1 jusqu'à 0,1. On peut ainsi mesurer l'acuité visuelle en fonction du contraste, en particulier en contraste faible.

Ce test permet de dépister une baisse d'acuité visuelle dans des conditions de faible niveau de contraste, qui est notamment constatée lors d'apparition d'une cataracte ou d'autres maladies de l'œil. Ce dispositif permet l'identification d'une baisse de performance en acuité visuelle.

Par ailleurs, le but d'une mesure d'acuité visuelle en vision mésopique est de quantifier la perte d'acuité visuelle en fonction du niveau d'éclairement. L'appareil de dépistage comporte avantageusement des moyens informatiques pour ajuster automatiquement le niveau de luminance, le niveau de contraste des optotypes et la couleur de fond de l'écran. Pour réaliser la mesure d'acuité visuelle en vision mésopique, on présente les optotypes en faisant varier le niveau de luminance et de contraste du fond de l'image affichée par l'écran, en partant des conditions de vision photopique, avec une luminance d'au moins 100 cd/m² et, au moyen d'une densité optique, diminuer la luminance jusqu'à des conditions de vision mésopique avec une luminance inférieure à environ 10 cd/m², et de préférence de l'ordre de 2 cd/m². De préférence, la modification des optotypes est réalisée en agissant sur deux paramètres de manière conjointe : modification de la luminance de l'écran (par exemple dans un écran à rétro-éclairage), ainsi que la modification du contraste de l'optotype affiché par rapport au fond de l'écran, par exemple en modifiant la couleur de fond. La modification de ces deux paramètres permet d'accroître la dynamique de la plage de luminance de l'écran.

Pour permettre d'avoir une luminance suffisante dans les différentes directions de visée, compte-tenu de la présence de la lame semi-réfléchissante sur le chemin optique entre la voie intermédiaire et la voie en vision de près ou en vision de loin, il est préférable de sélectionner un écran ayant une luminance égale au moins au double de la luminance d'au moins 100cd/m² et d'ajuster la luminance de l'écran en fonction du chemin optique. Ainsi, l'utilisateur perçoit des images ayant une luminance moyenne sensiblement identique quelle que soit la direction de visée.

La tablette comprend généralement une batterie ou des moyens de raccordement à une source de courant électrique.

L'appareil de dépistage 100 offre l'avantage d'être très économe en énergie car l'écran de la tablette est la principale source de consommation d'énergie, le système opto-mécanique à miroirs et lentilles étant fixe et entièrement passif.

L'écran est facilement interchangeable pour une mise à jour matérielle ou logicielle de l'appareil.

### Procédé

Sur la figure 3, on a représenté en vue de dessus un écran 30 d'une tablette tactile 3 intégrée dans un appareil de dépistage des défauts de la vue d'un utilisateur.

L'appareil de dépistage 100 incorpore un écran plat de type LCD équipé d'une dalle tactile. Cet écran est implanté au sein d'un appareil de sorte qu'une première zone de la surface de l'écran soit en vis-à-vis de voies optique à l'intérieur de l'appareil de dépistage et de sorte que la deuxième zone 32 de l'écran est apparente à l'extérieur de l'appareil de dépistage, cette deuxième zone 32 étant disposée à proximité du patient.

Les fonctions de l'écran sont avantageusement réalisées par une tablette tactile, mettant à profit l'électronique de commande de la tablette pour le pilotage de l'écran, la communication avec d'autres systèmes électroniques tels qu'une télécommande, un ordinateur ou imprimante pour l'édition et la sauvegarde d'un rapport de test de dépistage.

De manière complémentaire, des fonctions audio peuvent être mises en œuvreà des fins d'explication. En option, l'appareil de dépistage permet en outre un dépistage auditif.

La première zone 31 de l'écran n'est pas accessible à l'utilisateur, est commandée électroniquement et informatiquement de façon à présenter les optotypes souhaités.

La deuxième zone 32 de l'écran est accessible pour l'affichage d'informations et pour l'enregistrement des réponses de l'utilisateur par interaction tactile, au cours des tests. La deuxième zone 32 externe peut être utilisée comme écran d'accueil permettant la sélection du ou des tests souhaités par l'utilisateur. La deuxième zone 32 de l'écran assure les fonctions d'interface homme-machine entre l'utilisateur et l'appareil de dépistage. Par exemple, dans le cas d'une utilisation en autotest, la deuxième zone 32 externe peut afficher un mode d'emploi ou une invitation à dépister sa vue en totale autonomie.

De manière optionnelle, une troisième zone de l'écran, disposée aussi à l'extérieur du boîtier de l'appareil de dépistage permet d'afficher simultanément une duplication de l'optotype présenté sur la première zone. Cette troisième zone est avantageusement disposée face à un opérateur ou un médecin pour lui permettre de suivre en temps réel le déroulement de l'examen de dépistage.

L'utilisateur valide le démarrage d'un test de dépistage, par exemple, via un clic sur un bouton tactile sur la deuxième zone 32 de l'écran. Puis, il pose sa tête en appui contre la mentonnière 8 et/ou l'appui frontal. Ainsi son œil droit est face à une ouverture droite du boîtier de l'appareil et son œil gauche face à une ouverture gauche.

Pour un test de dépistage en vision stéréoscopique, l'image droite et l'image gauche sont activées, tandis que pour un test de dépistage en vision monoculaire, une seule image, droite ou gauche, est activée, le reste de l'écran étant noir.

Le système électronique et informatique de l'appareil est configuré pour afficher sur la première zone 31 de l'écran des tests à une distance ou une proximité prédéterminée (correspondant de préférence à la vision de près, de loin et/ou intermédiaire) et pour enregistrer directement les réponses du patient via la deuxième zone 32 externe de l'écran.

Les figures 6A-6B illustrent un exemple d'interface graphique pour un test basé sur des anneaux de Landolt.

De manière automatique ou suite à une interaction tactile de l'utilisateur sur la deuxième zone 32, on affiche un anneau de Landolt 50 sur la première zone 31 de l'écran, par exemple sur la première partie droite 301. L'anneau de Landolt 50 est affiché en couleur sombre sur fond clair, et de préférence en noir sur fond blanc. Le logiciel de commande ou l'utilisateur ajuste le diamètre de l'anneau de Landolt pour tester son acuité visuelle pour une taille déterminée de l'optotype et pour une distance de vision déterminée. L'anneau de Landolt 50 comporte une ouverture 53. Le logiciel génère un anneau de Landolt du diamètre déterminé, mais dont l'orientation de l'ouverture est sélectionnée aléatoirement parmi huit orientations: 0, +45 ,+90, +135, +180, +225, +270 et +315 degrés par rapport à une ligne verticale. Dans l'exemple de la figure 6, l'orientation de l'anneau de Landolt est de +45 degrés.

Le but du test est de déterminer si l'utilisateur a une acuité visuelle lui permettant de distinguer correctement l'orientation de l'anneau de Landolt.

Le procédé peut prévoir différentes variantes pour enregistrer la réponse de l'utilisateur via la deuxième zone 32 de l'écran tactile.

Selon une première variante illustrée sur les figures 6A et 6B, on affiche d'abord un anneau de Landolt, sur appui de l'utilisateur dans la zone 32, une flèche s'affiche (ou un autre repère graphique approprié) dans une position initiale 51 qui est indépendante de l'orientation de l'anneau de Landolt 50.

L'utilisateur regarde via l'ouverture sélectionnée et dans l'axe de visée approprié, l'image affichée sur la première zone de l'écran qui lui permet d'observer simultanément l'anneau de Landolt et la flèche.

Par des actions de glisser-déplacer sur la zone externe 32, l'utilisateur peut déplacer la flèche autour de l'anneau de Landolt, de préférence par pas de 1/8 de tour. Lorsque l'utilisateur estime visuellement sur l'image affichée que la flèche est disposée de manière à indiquer l'orientation de l'ouverture de l'anneau, il valide la position de la flèche 53, par exemple via un bouton tactile sur la deuxième zone 32 de l'écran. Le système informatique enregistre ainsi la réponse de l'utilisateur et analyse si cette réponse est correcte ou pas.

Le système peut enchaîner une série de plusieurs tests pour un même diamètre et différentes orientations de l'anneau de Landolt.

En fonction des réponses enregistrées, le système de commande modifie le diamètre de l'anneau de Landolt affiché, pour passer à un degré supérieur ou inférieur de l'échelle d'acuité visuelle.

Dans une autre variante, on affiche en superposition deux anneaux de Landolt de même diamètre. Le premier anneau de Landolt a une orientation fixée aléatoirement parmi les huit orientations. Au contraire, le deuxième anneau de Landolt a une orientation variable en fonction des interactions de l'utilisateur sur la deuxième zone : par des interactions sur la dalle tactile sur la deuxième zone 32 de l'écran tactile, l'utilisateur peut faire tourner le deuxième anneau de Landolt sur lui-même dans un sens ou dans l'autre tandis que le premier anneau de Landolt reste fixe. Lorsque l'orientation des deux anneaux de Landolt est différente l'une de l'autre, l'écran affiche un cercle complet, l'ouverture de chaque anneau de Landolt étant obturée respectivement par le cercle de l'autre anneau de Landolt. Lorsque l'orientation du deuxième anneau de Landolt coïncide avec l'orientation du premier anneau de Landolt, l'écran affiche un anneau de Landolt ayant l'orientation du premier anneau. Lorsque l'utilisateur estime visuellement sur l'image affichée qu'il voit l'image d'un anneau de Landolt ouvert et non pas un cercle fermé, il valide l'orientation du deuxième anneau, par exemple via un clic sur un bouton tactile de la deuxième zone 32 de l'écran.

Le système électronique et informatique enregistre ensuite la réponse de l'utilisateur.

Ces deux variantes de procédés pour déterminer l'orientation d'un anneau de Landolt sont basées essentiellement sur un affichage graphique et sur une saisie tactile. Ces deux variantes permettent d'éviter les faux positifs associés à la détermination de l'orientation d'un anneau de Landolt fondées une réponse orale ou écrite de l'utilisateur.

Toutefois, le système et le procédé de dépistage ne sont nullement limités à l'utilisation d'anneaux de Landolt. Ils sont aussi compatibles avec des tests de dépistage visuel basés sur la reconnaissance de caractères alphanumériques, tels qu'illustrés sur la figure 7.

La figure 7 représente une interface graphique affichant un symbole alphanumérique 54 sélectionné aléatoirement par le système informatique parmi une pluralité de symboles alphanumériques.

Dans un premier temps, on affiche une lettre 54 sur l'écran. Dans un deuxième temps, on affiche, par exemple sur la partie droite de l'image, un tableau 56, qui représente un ensemble de symboles alphanumériques, dont l'un est identique à celui affiché dans la partie gauche de l'écran 31.

Une flèche 55, ou tout autre repère graphique approprié, est affiché simultanément à proximité du tableau 56 de symboles alphanumériques. Par des interactions sur la deuxième zone 32 de l'écran, par exemple via des actions de glisser-déplacer de haut en bas, l'utilisateur peut déplacer la position de la flèche 55 dans l'image affichée, de manière à placer la flèche 55 face à l'un ou l'autre des symboles alphanumériques de la série 56. Lorsque l'utilisateur estime visuellement sur l'image affichée que la flèche 55 indique le même symbole alphanumérique dans la série 56 qu'au centre de l'image, il valide la position de la flèche, par exemple via un clic sur un bouton tactile de la deuxième zone 32 de l'écran.

L'utilisateur peut ainsi enregistrer, par des interactions tactiles sur la deuxième zone de l'écran 32, ses réponses à un test visuel affichés sur la première zone de l'écran, sans passer par des réponses faisant intervenir le langage oral ou écrit, ce qui permet d'éviter des erreurs de communication.

D'autres tests de dépistage peuvent être implémentés sur l'appareil de dépistage, tels qu'un test d'astigmatisme (test de parent), affichant des rayons d'un demi-arc de cercle et dans lequel un curseur de type graphique, par exemple à barres circulaires à l'extrémité des rayons, permet d'identifier le rayon perçu par le patient comme étant le plus contrasté. Le patient enregistre sa réponse au moyen de la dalle tactile de manière analogue à l'un des modes de réalisation décrits ci-dessus.

Les tests peuvent être effectués en vision monoculaire sur chaque œil séparément. D'autres tests peuvent être réalisés en vision stéréoscopique, chaque œil visualisant une image virtuelle différente formée suivant un chemin optique séparé et le patient fusionnant, ou pas, l'image virtuelle droite avec l'image virtuelle gauche.

Le procédé de dépistage permet ainsi de faire passer différents tests de dépistage visuels, notamment :
- mesure de la réserve d'accommodation ou punctum proximum ;
- test d'éblouissement ;
- mesure d'acuité visuelle avec échelle d'anneaux de Landolt ou échelle de caractères alphanumériques ;
- mesure d'acuité visuelle en contraste variable ;
- mesure d'acuité visuelle en vision mésopique ;
- test d'astigmatisme ;
- tendance hypermétropique ;
- tendance amétropique ;
- vision des couleurs ;
- vision des reliefs.

## Revendications

1. Appareil opto-électronique bi-oculaire de dépistage des défauts de la vue et de mesure d'acuité visuelle d'un utilisateur, l'appareil opto-électronique bi-oculaire (100) comportant :
- un boîtier (10) ayant une première ouverture droite (11) et une première ouverture gauche (21) destinées à être disposées respectivement face à l'œil droit (1) et à l'œil gauche (2) de l'utilisateur regardant dans une première direction de visée ;
**caractérisé en ce que** l'appareil opto-électronique bi-oculaire (100) comporte :
- un écran tactile (30) comprenant une première zone (31) et une deuxième zone (32) séparées spatialement,
- l'écran tactile (30) étant disposé dans l'appareil opto-électronique bi-oculaire (100) de manière à ce que la première zone (31) de l'écran tactile (30) soit à l'intérieur du boîtier (10) et de manière à ce que la deuxième zone (32) de l'écran tactile (30) soit accessible à l'extérieur du boîtier (10) ;
- des moyens électroniques adaptés pour afficher une pluralité d'optotypes sur une première partie (310, 301, 302) de la première zone (31) de l'écran tactile (30), interne au boîtier ;
- un système optique (41, 42, 43, 44, 45, 46, 47, 48) disposé à l'intérieur du boîtier (10), le système optique étant configuré (41, 42, 43, 44, 45, 46, 47, 48) pour former une première image droite de la première partie (310, 301, 302) de la première zone (31) de l'écran tactile (30) en direction de la première ouverture droite (11) à une première distance de vision suivant un premier chemin optique, et le système optique (41, 42, 43, 44, 45, 46, 47, 48) étant configuré pour former une première image gauche de la première partie (310, 301, 302) de la première zone (31) de l'écran tactile (30) en direction de la première ouverture gauche (21) à ladite première distance de vision suivant un deuxième chemin optique,
**caractérisé en ce que**
le boîtier (10) comporte une paroi externe (6) opaque qui est disposée transversalement à la surface de l'écran tactile (30) de manière à séparer physiquement et optiquement la première zone (31) de la deuxième zone (32).

2. Appareil opto-électronique bi-oculaire de dépistage des défauts de la vue et de mesure d'acuité visuelle d'un utilisateur selon la revendication 1, dans lequel la deuxième zone (32) de l'écran tactile (30) est configurée pour enregistrer des interactions tactiles de l'utilisateur et comportant en outre des moyens électroniques de traitement adaptés pour modifier l'affichage desdits optotypes sur la première partie (310, 301, 302) de la première zone (31) de l'écran en fonction des interactions tactiles enregistrées et/ou pour enregistrer une réponse de l'utilisateur à une première image droite visualisée et/ou à une première image gauche visualisée en fonction desdites interactions tactiles enregistrées.

3. Appareil opto-électronique bi-oculaire de dépistage des défauts de la vue et de mesure d'acuité visuelle d'un utilisateur selon la revendication 1 ou 2, comportant en outre :
- une deuxième ouverture droite (12) et une deuxième ouverture gauche (22) destinées à être disposées respectivement face à l'œil droit (1) et à l'œil gauche (2) de l'utilisateur regardant dans une deuxième direction de visée ;
- les moyens électroniques étant adaptés pour afficher une pluralité d'optotypes sur une deuxième partie (320, 321, 322) de la première zone (31) de l'écran tactile (30), interne au boîtier ; et
- le système optique (41, 42, 43, 44, 45, 46, 47, 48) étant configuré pour former une deuxième image droite de la deuxième partie (320, 321, 322) de la première zone (31) de l'écran tactile en direction de la deuxième ouverture droite (12) à une deuxième distance de vision suivant un troisième chemin optique, et le système optique (41, 42, 43, 44, 45, 46, 47, 48) étant configuré pour former une deuxième image gauche de la deuxième partie (320, 321, 322) de la première zone (31) de l'écran tactile en direction de la deuxième ouverture gauche (22) à ladite deuxième distance de vision suivant un quatrième chemin optique.

4. Appareil opto-électronique bi-oculaire de dépistage des défauts de la vue et de mesure d'acuité visuelle d'un utilisateur selon la revendication 3, comportant en outre :
- une troisième ouverture droite (13) et une troisième ouverture gauche (23) destinées à être disposées respectivement face à l'œil droit (1) et à l'œil gauche (2) de l'utilisateur regardant dans une troisième direction de visée ; et
- le système optique (41, 42, 43, 44, 45, 46, 47, 48) étant configuré pour former une troisième image droite d'une partie (301, 302) de la première zone (31) de l'écran tactile en direction de la troisième ouverture droite (13) à une troisième distance de vision suivant un cinquième chemin optique, et le système optique (41, 42, 43, 44, 45, 46, 47, 48) étant configuré pour former une troisième image gauche de la troisième partie (301, 302) de la première zone (31) de l'écran tactile en direction de la troisième ouverture gauche (23) à ladite troisième distance de vision suivant un sixième chemin optique.

5. Appareil opto-électronique bi-oculaire de dépistage des défauts de la vue et de mesure d'acuité visuelle d'un utilisateur selon l'une des revendications précédentes comportant en outre des moyens de synthèse vocale.

6. Appareil opto-électronique bi-oculaire de dépistage des défauts de la vue et de mesure d'acuité visuelle d'un utilisateur selon l'une des revendications précédentes dans lequel l'écran tactile (30) est disposé dans l'appareil opto-électronique bi-oculaire de manière à présenter une troisième zone externe destinée à servir d'interface à un opérateur, ladite troisième zone externe étant séparée spatialement desdites première zone (31) et deuxième zone (32) de l'écran tactile.

7. Appareil opto-électronique bi-oculaire de dépistage des défauts de la vue et de mesure d'acuité visuelle d'un utilisateur selon l'une des revendications précédentes comportant en outre des moyens d'obturation adaptés pour obturer sélectivement une première, deuxième et/ou troisième ouverture droite (11, 12, 13) et/ou une première, deuxième et/ou troisième ouverture gauche (21, 22, 23).

8. Appareil opto-électronique bi-oculaire de dépistage des défauts de la vue et de mesure d'acuité visuelle d'un utilisateur selon l'une des revendications précédentes dans lequel la pluralité d'optotypes comporte un anneau de Landolt (50) ayant une orientation et un diamètre déterminés et dans lequel les moyens de traitement sont adaptés pour indiquer l'orientation ou pour modifier l'orientation et/ou le diamètre de l'anneau de Landolt par interaction de l'utilisateur via la deuxième zone (32) externe de l'écran tactile (30).

9. Appareil opto-électronique bi-oculaire de dépistage des défauts de la vue et de mesure d'acuité visuelle d'un utilisateur selon l'une des revendications précédentes dans lequel la pluralité d'optotypes comporte un caractère alphanumérique (54) ayant une taille déterminée et dans lequel les moyens de traitement sont adaptés pour désigner un caractère alphanumérique parmi un ensemble (56) de caractères alphanumériques ou pour modifier le caractère alphanumérique (54) affiché et/ou la taille du caractère alphanumérique (54) affiché par interaction de l'utilisateur via la deuxième zone (32) externe de l'écran tactile (30).

10. Appareil opto-électronique bi-oculaire de dépistage des défauts de la vue et de mesure d'acuité visuelle d'un utilisateur selon la revendication 2 et l'une des revendications 1, 3 ou 4, dans lequel les moyens électroniques de traitement sont adaptés pour déduire de cet enregistrement une mesure d'acuité visuelle de l'œil droit et/ou de l'œil gauche pour la première distance de vision, respectivement pour la deuxième distance de vision et/ou pour la troisième distance de vision, la première distance de vision correspondant de préférence une distance de vision de près inférieure ou égale à 50 cm, la deuxième distance de vision correspondant de préférence une distance de vision de loin supérieure ou égale à 4 mètres, la troisième distance de vision correspondant de préférence une distance de vision intermédiaire comprise entre 50 cm et 3 m.

11. Appareil opto-électronique bi-oculaire de dépistage des défauts de la vue et de mesure d'acuité visuelle d'un utilisateur selon l'une des revendications précédentes comportant en outre des moyens de modification de la luminance et/ou du contraste des images affichées sur la première zone (31) de l'écran tactile.

12. Appareil opto-électronique bi-oculaire de dépistage des défauts de la vue et de mesure d'acuité visuelle d'un utilisateur selon l'une des revendications précédentes comportant en outre des moyens de mesure de la réserve d'accommodation de l'utilisateur comprenant un sous-ensemble de mesure de la réserve d'accommodation monté sur un dispositif d'escamotage, le sous-ensemble de mesure de la réserve d'accommodation comportant deux porte-test (57) mobiles en translation suivant des rails de guidage (67) disposés parallèlement à l'axe de vision intermédiaire.

13. Appareil opto-électronique bi-oculaire de dépistage des défauts de la vue et de mesure d'acuité visuelle d'un utilisateur selon la revendication 12 dans lequel l'écran tactile est adapté pour former un moyen d'éclairage adapté pour éclairer lesdits porte-tests du sous-ensemble de mesure de la réserve d'accommodation.

14. Appareil opto-électronique bi-oculaire de dépistage des défauts de la vue et de mesure d'acuité visuelle d'un utilisateur selon l'une des revendications précédentes, ledit appareil comprenant un logement dans lequel l'écran tactile (30) est inséré, ledit écran tactile étant amovible du boîtier de l'appareil.

15. Procédé de dépistage des défauts de la vue et de mesure d'acuité visuelle d'un utilisateur, le procédé mettant en oeuvre un appareil opto-électronique bi-oculaire selon l'une des revendications 1 à 14 et comprenant les étapes suivantes :
a. affichage d'une pluralité d'optotypes (50, 54) sur la première zone (31) de l'écran tactile, ladite pluralité d'optotypes (50, 54) comprenant au moins un anneau de Landolt (50), ayant un diamètre et une ouverture d'orientation prédéterminée ;
b. modification du diamètre et/ou d'une indication de l'orientation de l'ouverture de l'anneau de Landolt détectée visuellement par interaction via la deuxième zone (32) de l'écran tactile ;
c. validation, via la deuxième zone (32) de l'écran tactile, d'un diamètre et/ou d'une indication de l'orientation de l'anneau de Landolt détectée visuellement par l'utilisateur.

16. Procédé de dépistage des défauts de la vue et de mesure d'acuité visuelle d'un utilisateur, le procédé mettant en œuvreun appareil opto-électronique bi-oculaire selon l'une des revendications 1 à 14 et comprenant les étapes suivantes :
a. affichage d'une pluralité d'optotypes (50, 54) sur la première zone (31) de l'écran tactile, ladite pluralité d'optotypes (50, 54) comprenant au moins un caractère alphanumérique (54) ayant une taille prédéterminée ;
b. modification du caractère alphanumérique (54) affiché, de la taille du caractère alphanumérique (54) affiché et/ou d'une indication (55) du caractère alphanumérique (54) par interaction via la deuxième zone (32) de l'écran tactile ;
c. validation par l'utilisateur, via la deuxième zone (32) de l'écran tactile, d'une taille et/ou d'une indication (55) du caractère alphanumérique (54) affiché.

## Patentansprüche

1. Binokulares optoelektronisches Gerät zum Screening von Sehfehlern und zum Messen der Sehschärfe eines Benutzers, wobei das binokulare optoelektronische Gerät (100) umfasst:
- ein Gehäuse (10) mit einer ersten rechten Öffnung (11) und einer ersten linken Öffnung (21), die dazu bestimmt sind, gegenüber dem rechten Auge (1) bzw. dem linken Auge (2) des Benutzers angeordnet zu werden, der in eine erste Blickrichtung schaut;
**dadurch gekennzeichnet, dass** das binokulare optoelektronische Gerät (100) umfasst:
- einen Touchscreen (30) mit einem ersten Bereich (31) und einem zweiten Bereich (32), die räumlich getrennt sind,
- wobei der Touchscreen (30) in dem binokularen optoelektronischen Gerät (100) derart angeordnet ist, dass der erste Bereich (31) des Touchscreens (30) innerhalb des Gehäuses (10) ist, und derart, dass der zweite Bereich (32) des Touchscreens (30) außerhalb des Gehäuses (10) zugänglich ist;
- elektronische Mittel, die geeignet sind, eine Vielzahl von Sehzeichen auf einem ersten Teil (310, 301, 302) des ersten Bereichs (31) des Touchscreens (30) anzuzeigen, der sich im Gehäuse befindet;
- ein optisches System (41, 42, 43, 44, 45, 46, 47, 48), das im Innern des Gehäuses (10) angeordnet ist, wobei das optische System so ausgestaltet (41, 42, 43, 44, 45, 46, 47, 48) ist, dass es ein erstes rechtes Bild des ersten Teils (310, 301, 302) des ersten Bereichs (31) des Touchscreens (30) in Richtung der ersten rechten Öffnung (11) in einem ersten Sehabstand entlang eines ersten optischen Pfades bildet, und wobei das optische System (41, 42, 43, 44, 45, 46, 47, 48) so ausgestaltet ist, dass es ein erstes linkes Bild des ersten Teils (310, 301, 302) des ersten Bereichs (31) des Touchscreens (30) in Richtung der ersten linken Öffnung (21) in dem ersten Sehabstand entlang eines zweiten optischen Pfades bildet,
**dadurch gekennzeichnet, dass** das Gehäuse (10) eine undurchsichtige Außenwand (6) umfasst, die quer zur Oberfläche des Touchscreens (30) derart angeordnet ist, dass der erste Bereich (31) physisch und optisch vom zweiten Bereich (32) getrennt ist.

2. Binokulares optoelektronisches Gerät zum Screening von Sehfehlern und zum Messen der Sehschärfe eines Benutzers nach Anspruch 1, bei dem der zweite Bereich (32) des Touchscreens (30) dazu ausgestaltet ist, taktile Interaktionen des Benutzers aufzuzeichnen, und ferner elektronische Verarbeitungsmittel umfasst, die geeignet sind, die Anzeige der Sehzeichen auf dem ersten Teil (310, 301, 302) des ersten Bereichs (31) des Bildschirms in Abhängigkeit von den aufgezeichneten taktilen Interaktionen zu ändern und/oder eine Antwort des Benutzers auf ein visualisiertes erstes rechtes Bild und/oder auf ein visualisiertes erstes linkes Bild in Abhängigkeit von den aufgezeichneten taktilen Interaktionen aufzuzeichnen.

3. Binokulares optoelektronisches Gerät zum Screening von Sehfehlern und zum Messen der Sehschärfe eines Benutzers nach Anspruch 1 oder 2, umfassend ferner:
- eine zweite rechte Öffnung (12) und eine zweite linke Öffnung (22), die dazu bestimmt sind, gegenüber dem rechten Auge (1) bzw. dem linken Auge (2) des Benutzers angeordnet zu werden, der in eine zweite Blickrichtung schaut;
- wobei die elektronischen Mittel geeignet sind, eine Vielzahl von Sehzeichen auf einem zweiten Teil (320, 321, 322) des ersten Bereichs (31) des Touchscreens (30) anzuzeigen, der sich im Gehäuse befindet, und
- wobei das optische System (41, 42, 43, 44, 45, 46, 47, 48) so ausgestaltet ist, dass es ein zweites rechtes Bild des zweiten Teils (320, 321, 322) des ersten Bereichs (31) des Touchscreens in Richtung der zweiten rechten Öffnung (12) in einem zweiten Sehabstand entlang eines dritten optischen Pfades bildet, und wobei das optische System (41, 42, 43, 44, 45, 46, 47, 48) so ausgestaltet ist, dass es ein zweites linkes Bild des zweiten Teils (320, 321, 322) des ersten Bereichs (31) des Touchscreens in Richtung der zweiten linken Öffnung (22) in dem zweiten Sehabstand entlang eines vierten optischen Pfades bildet.

4. Binokulares optoelektronisches Gerät zum Screening von Sehfehlern und zum Messen der Sehschärfe eines Benutzers nach Anspruch 3, umfassend ferner:
- eine dritte rechte Öffnung (13) und eine dritte linke Öffnung (23), die dazu bestimmt sind, gegenüber dem rechten Auge (1) bzw. dem linken Auge (2) des Benutzers angeordnet zu werden, der in eine dritte Blickrichtung schaut, und
- wobei das optische System (41, 42, 43, 44, 45, 46, 47, 48) so ausgestaltet ist, dass es ein drittes rechtes Bild eines Teils (301, 302) des ersten Bereichs (31) des Touchscreens in Richtung der dritten rechten Öffnung (13) in einem dritten Sehabstand entlang eines fünften optischen Pfades bildet, und wobei das optische System (41, 42, 43, 44, 45, 46, 47, 48) so ausgestaltet ist, dass es ein drittes linkes Bild des dritten Teils (301, 302) des ersten Bereichs (31) des Touchscreens in Richtung der dritten linken Öffnung (23) in dem dritten Sehabstand entlang eines sechsten optischen Pfades bildet.

5. Binokulares optoelektronisches Gerät zum Screening von Sehfehlern und zum Messen der Sehschärfe eines Benutzers nach einem der vorhergehenden Ansprüche, umfassend ferner Sprachsynthesemittel.

6. Binokulares optoelektronisches Gerät zum Screening von Sehfehlern und zum Messen der Sehschärfe eines Benutzers nach einem der vorhergehenden Ansprüche, bei dem der Touchscreen (30) in dem optoelektronischen binokularen Gerät derart angeordnet ist, dass er einen dritten äußeren Bereich aufweist, der dazu bestimmt ist, als Schnittstelle für einen Bediener zu dienen, wobei der dritte äußere Bereich von dem ersten Bereich (31) und dem zweiten Bereich (32) des Touchscreens räumlich getrennt ist.

7. Binokulares optoelektronisches Gerät zum Screening von Sehfehlern und zum Messen der Sehschärfe eines Benutzers nach einem der vorhergehenden Ansprüche, umfassend ferner Verschließmittel, die geeignet sind, eine erste, zweite und/oder dritte rechte Öffnung (11, 12, 13) und/oder eine erste, zweite und/oder dritte linke Öffnung (21, 22, 23) selektiv zu verschließen.

8. Binokulares optoelektronisches Gerät zum Screening von Sehfehlern und zum Messen der Sehschärfe eines Benutzers nach einem der vorhergehenden Ansprüche, bei dem die Vielzahl von Sehzeichen einen Landolt-Ring (50) umfasst, der eine bestimmte Ausrichtung und einen bestimmten Durchmesser hat, und bei dem die Verarbeitungsmittel geeignet sind, durch Interaktion des Benutzers über den zweiten äußeren Bereich (32) des Touchscreens (30) die Ausrichtung anzugeben und/oder die Ausrichtung und/oder den Durchmesser des Landolt-Rings zu ändern.

9. Binokulares optoelektronisches Gerät zum Screening von Sehfehlern und zum Messen der Sehschärfe eines Benutzers nach einem der vorhergehenden Ansprüche, bei dem die Vielzahl von Sehzeichen ein alphanumerisches Zeichen (54) mit einer bestimmten Größe umfasst und bei dem die Verarbeitungsmittel geeignet sind, durch Interaktion des Benutzers über den zweiten äußeren Bereich (32) des Touchscreens (30) ein alphanumerisches Zeichen unter einer Menge (56) von alphanumerischen Zeichen zu bezeichnen oder das angezeigte alphanumerische Zeichen (54) und/oder die Größe des angezeigten alphanumerischen Zeichens (54) zu ändern.

10. Binokulares optoelektronisches Gerät zum Screening von Sehfehlern und zum Messen der Sehschärfe eines Benutzers nach Anspruch 2 und einem der Ansprüche 1, 3 oder 4, bei dem die elektronischen Verarbeitungsmittel geeignet sind, aus dieser Aufzeichnung eine Sehschärfemessung des rechten Auges und/oder des linken Auges für den ersten Sehabstand bzw. für den zweiten Sehabstand und/oder für den dritten Sehabstand abzuleiten, wobei der erste Sehabstand bevorzugt einem Sehabstand von knapp unter oder gleich 50 cm entspricht, der zweite Sehabstand bevorzugt einem Sehabstand von weit über oder gleich 4 m entspricht, der dritte Sehabstand bevorzugt einem Zwischensehabstand zwischen 50 cm und 3 m entspricht.

11. Binokulares optoelektronisches Gerät zum Screening von Sehfehlern und zum Messen der Sehschärfe eines Benutzers nach einem der vorhergehenden Ansprüche, umfassend ferner Mittel zum Ändern der Leuchtdichte und/oder des Kontrastes der auf dem ersten Bereich (31) des Touchscreens angezeigten Bilder.

12. Binokulares optoelektronisches Gerät zum Screening von Sehfehlern und zum Messen der Sehschärfe eines Benutzers nach einem der vorhergehenden Ansprüche, umfassend ferner Mittel zum Messen der Akkommodationsreserve des Benutzers, umfassend eine Baugruppe zum Messen der Akkommodationsreserve, die an einer Einklappvorrichtung montiert ist, wobei die Baugruppe zum Messen der Akkommodationsreserve zwei Testträger (57) umfasst, die entlang von parallel zur Zwischensehachse angeordneten Führungsschienen (67) translatorisch beweglich sind.

13. Binokulares optoelektronisches Gerät zum Screening von Sehfehlern und zum Messen der Sehschärfe eines Benutzers nach Anspruch 12, bei dem der Touchscreen geeignet ist, ein Beleuchtungsmittel zu bilden, das geeignet ist, die Testträger der Baugruppe zum Messen der Akkommodationsreserve zu beleuchten.

14. Binokulares optoelektronisches Gerät zum Screening von Sehfehlern und zum Messen der Sehschärfe eines Benutzers nach einem der vorhergehenden Ansprüche, wobei das Gerät eine Aufnahme umfasst, in die der Touchscreen (30) eingesetzt wird, wobei der Touchscreen aus dem Gehäuse des Geräts entnehmbar ist.

15. Verfahren zum Screening der Sehfehler und zum Messen der Sehschärfe eines Benutzers, wobei das Verfahren ein binokulares optoelektronisches Gerät nach einem der Ansprüche 1 bis 14 einsetzt und die folgenden Schritte umfasst:
a. Anzeigen einer Vielzahl von Sehzeichen (50, 54) auf dem ersten Bereich (31) des Touchscreens, wobei die Vielzahl von Sehzeichen (50, 54) mindestens einen Landolt-Ring (50) umfasst, der einen Durchmesser und eine Öffnung mit vorbestimmter Ausrichtung hat;
b. Ändern des Durchmessers und/oder einer Angabe der Ausrichtung der Öffnung des Landolt-Rings, die optisch erfasst wird, durch Interaktion über den zweiten Bereich (32) des Touchscreens;
c. Bestätigen, über den zweiten Bereich (32) des Touchscreens, eines Durchmessers und/oder einer Angabe der Ausrichtung des Landolt-Rings, die vom Benutzer optisch erfasst wird.

16. Verfahren zum Screening der Sehfehler und zum Messen der Sehschärfe eines Benutzers, wobei das Verfahren ein binokulares optoelektronisches Gerät nach einem der Ansprüche 1 bis 14 einsetzt und die folgenden Schritte umfasst:
a. Anzeigen einer Vielzahl von Sehzeichen (50, 54) auf dem ersten Bereich (31) des Touchscreens, wobei die Vielzahl von Sehzeichen (50, 54) mindestens ein alphanumerisches Zeichen (54) mit einer vorbestimmten Größe umfasst;
b. Ändern des angezeigten alphanumerischen Zeichens (54), der Größe des angezeigten alphanumerischen Zeichens (54) und/oder einer Angabe (55) des alphanumerischen Zeichens (54) durch Interaktion über den zweiten Bereich (32) des Touchscreens;
c. Bestätigen durch den Benutzer, über den zweiten Bereich (32) des Touchscreens, einer Größe und/oder einer Angabe (55) des angezeigten alphanumerischen Zeichens (54).

## Claims

1. A bi-ocular optoelectronic apparatus for screening for defects in the sight of and for measuring the visual acuity of a user, the bi-ocular optoelectronic apparatus (100) including:
- a housing (10) having a first right aperture (11) and a first left aperture (21), which apertures are respectively intended to be placed facing the right eye (1) and the left eye (2) of the user facing in a first direction of sight; **characterized in that** the bi-ocular optoelectronic apparatus (100) includes:
- a touchscreen (30) comprising a first zone (31) and a second zone (32) that are spatially separate,
- the touchscreen (30) being placed in the bi-ocular optoelectronic apparatus (100) so that the first zone (31) of the touchscreen (30) is in the interior of the housing (10) and so that the second zone (32) of the touchscreen (30) is accessible from the exterior of the housing (10);
- electronic means suitable for displaying a plurality of optotypes in a first portion (310, 301, 302) of the first zone (31) of the touchscreen (30), internal to the housing;
- an optical system (41, 42, 43, 44, 45, 46, 47, 48) placed in the interior of the housing (10), the optical system (41, 42, 43, 44, 45, 46, 47, 48) being configured to form a first right image of the first portion (310, 301, 302) of the first zone (31) of the touchscreen (30) in the direction of the first right aperture (11) at a first vision distance following a first optical path, and the optical system (41, 42, 43, 44, 45, 46, 47, 48) being configured to form a first left image of the first portion (310, 301, 302) of the first zone (31) of the touchscreen (30) in the direction of the first left aperture (21) at said first vision distance following a second optical path,
**characterized in that**
- the housing (10) includes an opaque external wall (6) that is placed transversely to the surface of the touchscreen (30) so as to separate physically and optically the first zone (31) from the second zone (32).

2. The bi-ocular optoelectronic apparatus for screening for defects in the sight of and for measuring the visual acuity of a user as claimed in claim 1, wherein the second zone (32) of the touchscreen (30) is configured to record touch interactions of the user and furthermore including electronic processing means suitable for modifying the display of said optotypes in the first portion (310, 301, 302) of the first zone (31) of the screen depending on the recorded touch interactions and/or to record a response of the user to a viewed first right image and/or to a viewed first left image depending on said recorded touch interactions.

3. The bi-ocular optoelectronic apparatus for screening for defects in the sight of and for measuring the visual acuity of a user as claimed in claim 1 or 2, furthermore including:
- a second right aperture (12) and a second left aperture (22), which apertures are respectively intended to be placed facing the right eye (1) and the left eye (2) of the user facing in a second direction of sight;
- the electronic means being suitable for displaying a plurality of optotypes in a second portion (320, 321, 322) of the first zone (31) of the touchscreen (30), internal to the housing; and
- the optical system (41, 42, 43, 44, 45, 46, 47, 48) being configured to form a second right image of the second portion (320, 321, 322) of the first zone (31) of the touchscreen in the direction of the second right aperture (12) at a second vision distance following a third optical path, and the optical system (41, 42, 43, 44, 45, 46, 47, 48) being configured to form a second left image of the second portion (320, 321, 322) of the first zone (31) of the touchscreen in the direction of the second left aperture (22) at said second vision distance following a fourth optical path.

4. The bi-ocular optoelectronic apparatus for screening for defects in the sight of and for measuring the visual acuity of a user as claimed in claim 3, furthermore including:
- a third right aperture (13) and a third left aperture (23), which apertures are respectively intended to be placed facing the right eye (1) and the left eye (2) of the user facing in a third direction of sight; and
- the optical system (41, 42, 43, 44, 45, 46, 47, 48) being configured to form a third right image of a portion (301, 302) of the first zone (31) of the touchscreen in the direction of the third right aperture (13) at a third vision distance following a fifth optical path, and the optical system (41, 42, 43, 44, 45, 46, 47, 48) being configured to form a third left image of the third portion (301, 302) of the first zone (31) of the touchscreen in the direction of the third left aperture (23) at said third vision distance following a sixth optical path.

5. The bi-ocular optoelectronic apparatus for screening for defects in the sight of and for measuring the visual acuity of a user as claimed in one of the preceding claims, furthermore including speech synthesizing means.

6. The bi-ocular optoelectronic apparatus for screening for defects in the sight of and for measuring the visual acuity of a user as claimed in one of the preceding claims wherein the touchscreen (30) is placed in the bi-ocular optoelectronic apparatus so as to have an external third zone intended to serve as an operator interface, said external third zone being spatially separate from said first zone (31) and second zone (32) of the touchscreen.

7. The bi-ocular optoelectronic apparatus for screening for defects in the sight of and for measuring the visual acuity of a user as claimed in one of the preceding claims furthermore including obturating means suitable for selectively obturating a first, second and/or third right aperture (11, 12, 13) and/or a first, second and/or third left aperture (21, 22, 23).

8. The bi-ocular optoelectronic apparatus for screening for defects in the sight of and for measuring the visual acuity of a user as claimed in one of the preceding claims wherein the plurality of optotypes includes a Landolt ring (50) having a determined orientation and diameter and wherein the processing means are suitable for indicating the orientation or for modifying the orientation and/or the diameter of the Landolt ring by interaction of the user via the external second zone (32) of the touchscreen (30).

9. The bi-ocular optoelectronic apparatus for screening for defects in the sight of and for measuring the visual acuity of a user as claimed in one of the preceding claims wherein the plurality of optotypes includes an alphanumeric character (54) having a determined size and wherein the processing means are suitable for designating an alphanumeric character from a set (56) of alphanumeric characters or for modifying the displayed alphanumeric character (54) and/or the size of the displayed alphanumeric character (54) by interaction of the user via the external second zone (32) of the touchscreen (30).

10. The bi-ocular optoelectronic apparatus for screening for defects in the sight of and for measuring the visual acuity of a user as claimed in claim 2 and one of claims 1, 3 and 4, wherein the electronic processing means are suitable for deducing from this recording a measurement of the visual acuity of the right eye and/or of the left eye for the first vision distance, for the second vision distance and/or for the third vision distance, respectively, the first vision distance preferably corresponding to a near-vision distance smaller than or equal to 50 cm, the second vision distance preferably corresponding to a far-vision distance larger than or equal to 4 meters and the third vision distance preferably corresponding to an intermediate-vision distance comprised between 50 cm and 3 m.

11. The bi-ocular optoelectronic apparatus for screening for defects in the sight of and for measuring the visual acuity of a user as claimed in one of the preceding claims furthermore including means for modifying the luminance and/or contrast of the images displayed in the first zone (31) of the touchscreen.

12. The bi-ocular optoelectronic apparatus for screening for defects in the sight of and for measuring the visual acuity of a user as claimed in one of the preceding claims, furthermore including means for measuring reserve accommodation of the user comprising a subassembly for measuring reserve accommodation mounted on a retraction device, the subassembly for measuring reserve accommodation comprising two test holders that are translatably movable along guiding rails (67) arranged parallel to the axis of intermediate vision.

13. The bi-ocular optoelectronic apparatus for screening for defects in the sight of and for measuring the visual acuity of a user as claimed in claim 12, wherein the touchscreen is adapted for forming an illuminating means suitable for illuminating said test holders of the subassembly for measuring reserve accommodation.

14. The bi-ocular optoelectronic apparatus for screening for defects in the sight of and for measuring the visual acuity of a user as claimed in one of the preceding claims, said apparatus comprising a hole wherein the touchscreen (30) is inserted, said touchscreen (30) being removable from the housing of the apparatus.

15. A method for screening for defects in the sight of and for measuring the visual acuity of a user, the method implementing a bi-ocular optoelectronic apparatus as claimed in one of claims 1 to 14 and comprising the following steps:
a. displaying a plurality of optotypes (50, 54) in the first zone (31) of the touchscreen, said plurality of optotypes (50, 54) comprising at least one Landolt ring (50), having a diameter and an aperture of predetermined orientation;
b. modifying the diameter and/or a visually detected indication of the orientation of the aperture of the Landolt ring by interaction via the second zone (32) of the touchscreen;
c. validating, via the second zone (32) of the touchscreen, a diameter and/or a visually detected indication of the orientation of the Landolt ring, this validation being by the user.

16. A method for screening for defects in the sight of and for measuring the visual acuity of a user, the method implementing a bi-ocular optoelectronic apparatus as claimed in one of claims 1 to 14 and comprising the following steps:
a. displaying a plurality of optotypes (50, 54) in the first zone (31) of the touchscreen, said plurality of optotypes (50, 54) comprising at least one alphanumeric character (54), having a predetermined size;
b. modifying the displayed alphanumeric character (54), the size of the displayed alphanumeric character (54) and/or an indication (55) of the alphanumeric character (54) by interaction via the second zone (32) of the touchscreen;
c. validating, via the second zone (32) of the touchscreen, a size and/or an indication (55) of the displayed alphanumeric character (54), this validation being by the user.
